# EUROPEAN PATENT SPECIFICATION

(11) **EP 4 243 698 B1**
(45) Date of publication and mention of the grant of the patent: **20.08.2025**
(21) Application number: 20807692.7
(22) Date of filing: 12.11.2020
(51) Int. Cl.: A61B 17/00, A61B 17/04

(54) **MINIMALLY INVASIVE FIXATION DEVICES FOR FIXATING A FELTABLE TEXTILE AND SET COMPRISING A FIXATION DEVICE**
MINIMALINVASIVE FIXIERVORRICHTUNGEN ZUM FIXIEREN EINES FILZFÄHIGEN TEXTILS UND SET MIT EINER FIXIERVORRICHTUNG
DISPOSITIFS DE FIXATION MINIMALEMENT INVASIFS POUR FIXER UN TEXTILE FEUTRABLE ET ENSEMBLE COMPRENANT UN DISPOSITIF DE FIXATION

(43) Date of publication of application: 20.09.2023
(73) Proprietor: ZuriMED Technologies AG, 8008 Zürich (CH)
(72) Inventor: BACHMANN, Elias, 8008 Zürich (CH); SAETHRE, Renée, 8008 Zürich (CH); LI, Xiang, 8008 Zürich (CH); WEBER, Alberto, 8008 Zürich (CH)
(74) Representative: Vossius & Partner Patentanwälte Rechtsanwälte mbB
(86) International application number: PCT/EP2020/081881
(87) International publication number: WO 2022/100832

(56) References cited:
- EP-A1- 2 455 037
- EP-A1- 3 132 811
- WO-A1-2016/044762
- CN-A- 109 137 267

## Description

The present invention is directed towards minimally invasive fixation devices for fixating a feltable textile, sets comprising a fixation device and methods for fixating a feltable textile at a target site in a patient.

Although there has been significant progress in the treatment of cardiovascular diseases, globally they constitute the leading cause of deaths, according to the World Health Organization (WHO). 50% of the population in first world countries die of cardiovascular diseases, but alongside economic growth, the prevalence in developing countries is rising, too. Consequently, an increasing number of people are in need of an invasive cardiovascular intervention every year.

Neonates with a ventricular septal defect (VSD) are born with a hole in the wall of the heart (septum) that separates the two lower chambers (ventricles). VSD is the most common heart defect and occurs when an infant's septum does not fully develop during pregnancy. The exact cause is unknown. A recent study by the Centers for Disease Control and Prevention in Atlanta estimated that 42 of every 10,000 infants are born with a VSD.

In a healthy human heart, the right side of the heart pumps blood to the lungs where the blood picks up oxygen and the left side of the heart then pumps the oxygen-rich blood to the rest of the body. In babies with VSD, blood that should be on the left side of the heart leaks into the right side. Hence, all of that extra blood gets pumped back into the lungs. This forces the heart and lungs to work harder, which can increase the infant's risk for other medical problems. Typically, VSD is treated by closing the hole with an implant. In some techniques, VSD is treated using open-heart surgery.

In general, open-heart surgery usually requires an incision along the sternum (median sternotomy), followed by cardiopulmonary bypass and the temporary arrest of the heart of the patient (cardioplegia), so that the operation can be done in a still environment without blood flowing. Such highly invasive techniques are not suitable for patients considered high risk due to their age, fragility or medical condition, as they involve a long recovery period and risks of trauma and infection. Therefore, in the past decade, many less invasive procedures for a variety of cardiovascular indications were developed, where the heart is accessed via a catheter through blood vessels (catheter-based cardiac surgery) or through very small incisions in the chest (minimally invasive cardiac surgery, MICS). These techniques are recently being expanded to also treat intermediate and low risk patients.

Many interventions depend on some kind of implant, e.g. heart valve replacements, annuloplasty rings, vascular grafts or septum defect repairs. Often, they are sutured to the human tissue to fix them into place, which must be done in a very careful manner by a well-trained surgeon in order to get the desired outcome. Placing the sutures adequately makes up a significant proportion of the operation time and the execution can sometimes be challenging or even impossible. Additionally, sutures can cut through, open up or loosen, or knot slippage may occur. Further, sutures have the disadvantage of a high application of stress concentrated where they are placed.

One example of an attachment mechanism is disclosed in EP 3 132 811 A1. EP 3 132 811 A1 discloses a staple cartridge for stapling tissue. However, stapling has the disadvantage that relatively high forces are applied and tissues are damaged. Further, a distribution of the attachment force is concentrated around the staple leading to undesirable force transmission.

WO 95/32756 discloses a catheter for retrograde perfusion of the heart through the coronary sinus. The catheter includes an inflatable balloon with retention means. The inflatable balloon includes spikes, a felt or a coating on the surface of the balloon as retention means that keep the balloon firmly in space. Though this document discloses a balloon as retention means, the retention is lost as soon as the balloon is deflated.

WO 2016/044762 A1 describes apparatus and methods for fabricating tubular structures from a combination of fibrous materials for use in tissue engineering scaffold applications. The fiber scaffolds may include a nonwoven felt that is attached to a further nonwoven felt with an attachment device including a plurality of needles. Thereby, the fibers of the felts are entangled with each other.

EP 2 455 037 A1 discloses an apparatus for treating a patent foramen ovale. The apparatus comprises an elongate catheter body having a proximal end and a distal end, an energy transmission element located on a distal portion of the catheter body for transmitting energy to contact tissue of the patent foramen ovale to close the patent foramen ovale, and a backstop element adapted to be positioned distal of the energy transmission element for facilitating energy delivery to the patent foramen ovale.

The problem of the present invention is providing devices and methods for attaching implants to a target site within the body in a minimally invasive fashion. In particular, it is an object of the invention to attach implants (e.g. cardiovascular implants) to the human tissue in a strong and durable fashion, while also simplifying the surgical procedure and therefore saving critical time, which would allow for a safer and more efficient intervention. A further optional object of the present invention is attaching the implant in a uniform manner allowing a uniform stress distribution of forces that are transmitted from and to the implant at the target site.

According to the invention the problem is solved with a minimally invasive fixation device for fixating a feltable textile according to claim 1 and a set comprising a fixation device according to claim 15. Optional embodiments are described in the dependent claims.

In one aspect, it is suggested to provide a minimally invasive fixation device for fixating a feltable textile at a target site. The feltable material is fixated at a target site in a human or in an animal. Preferable the target site comprises soft tissue. The fixation device comprises an elongate tubular member, a needle and a drive assembly. The elongate tubular member has a proximal end and a distal end and an interior lumen extending between the proximal end and the distal end. The elongate tubular member may be at least partially flexible. Preferably, the elongate tubular member is flexible over its entire length.

A textile as mentioned herein may be understood as a material made of fibers, filaments, or yarn. Flexible as mentioned herein may be understood in that the elongate tubular member may be bent, in particular while navigating a twisting path of a hollow organ (e.g. a blood vessel) and/or is capable of being brought into a bent shape. Elongate as mentioned herein might be understood to mean that the tubular member has a longer length than width (e.g. diameter). Preferably the elongate tubular member is flexible over its entire length. Alternatively, the elongate tubular member may have one or more flexible section(s). In some embodiments "elongate" may be understood as the tubular member having a length which is at least 5 times the width. The interior lumen may include a distal opening and a proximal opening.

The needle assembly comprises at least one felting needle, which is arranged or arrangeable at the distal end of the elongate tubular member. The at least one felting needle is movable relatively to the elongate tubular member, wherein the at least one felting needle is movable with a reciprocal motion. Reciprocal as mentioned herein may be understood as a back and forth motion, in particular a linear or circular back and forth motion. The needle assembly may comprise 2, 3, 4, 5, 6, 7, 8, 10 or more needles. The more felting needles the needle assembly comprises, the less reciprocal motions need to be made for a sufficiently strong attachment between the feltable textile and the target site.

The needle assembly can felt the feltable textile with fibers of the body by interweaving animal or human soft tissue fibers with fibers of the textile by pushing fibers from one into the other. If this is done repeatedly, a strong mechanical connection is achieved. The needle, its shape and the mechanism are explained in detail in the application PCT/CH2019/000015 by the applicant ZuriMED Technologies AG.

The drive assembly is arranged in the interior lumen of the tubular member and operably connected to the needle assembly for moving the at least one felting needle with the reciprocal motion when the at least one needle is located at the distal end such that the feltable textile can be fixated at the target site. The drive assembly may be fixedly attached to the elongate tubular member or movable relatively to the elongate tubular member. For example, the drive assembly may comprise a wire that extends through the lumen of the elongate tubular member. In some embodiments the drive assembly is arranged fully within the interior lumen while in other embodiments only parts of the drive assembly are arranged in the inner lumen.

Additionally, the fixation device may comprise an actuator or motor for actuating the drive assembly. The actuator may be an electrical, mechanical, hydraulic and/or pneumatic motor. The at least one felting needle may be driven by a pneumatic or hydraulic or electromagnetic actuator or motor. The actuators may be arranged within or outside of the elongate tubular member. The actuators may be arranged or are arrangeable at a distal end or operable connected to a distal end of the drive assembly. The actuator may by be arranged in a proximal or distal part of the fixation device. In some embodiments the actuator may be arranged in a distal portion of the fixation device, such that the reciprocal motion does not need to be transferred from the proximal end of the elongate tubular member to the at least one felting needle.

The fixation device provides a minimal invasive device and allows a deployment, positioning and fixing a feltable textile onto human or animal tissue. This device provides a reliable fixation while at the same time minimizing navigation and multi degrees of freedom problems which can occur when suturing in locations which are difficult to reach.

The elongate tubular member may have a diameter in the range of 2 Fr to 30 Fr, more specifically 4 Fr to 26 Fr, more specifically 12 to 22 Fr. These ranges correspond to suitable sizes for an atrial or venous access towards the heart.

As used herein the terms proximal and distal are defined from the perspective of a user of the percutaneous fixation device, in particular from the perspective of a medical professional such as a surgeon using the device.

The feltable textile may comprise or consist of absorbable fibers, particularly polyglycolic acid, polylactic acid, polydioxanone, or caprolactone. As mentioned herein, the term absorbable may relate to materials which are degraded after implantation in a human or animal body.

In some embodiments, the feltable textile comprises or consists of non-absorbable fibers, particularly silk, polypropylene, polyester or polyamide. As mentioned herein, the term non-absorbable relates to materials which are not degraded after implantation in a human or animal body.

In some embodiments, the feltable textile comprises or consists of a combination of absorbable fibers, particularly polyglycolic acid, polylactic acid, polydioxanone, or caprolactone and non-absorbable fibers, particularly silk, polypropylene, polyester or polyamide.

In certain embodiments, the fibers of the feltable textile comprise or consist of a biocompatible polymer, particularly polyethylene (PE, CAS No. 9002-88-4) polytetrafluorethylene (PTFE, CAS No. 9002-84-0), polyethylene terephthalate (PET, CAS No. 25038-59-9), poly(glycolide) (PGA, CAS No. 26124-68-5), poly(lactic-co-glycolic acid) (PLGA, CAS No. 26780-50-7), poly (L-lactic acid) (PLLA, CAS No. 33135-50-1), polycaprolactone (PCL, CAS No. 24980-41-4), 1,2-propanediol (PDO, CAS 57-55-6) or 1,3-propanediol (PDO, CAS No. 504-63-2).

In certain embodiments, the feltable textile could also be made of decellularized collagen tissue from human or animal origin.

In a preferred embodiment, the drive assembly is adapted to drive the reciprocal motion at a predetermined frequency. The frequency is preferably in a range from 1 Hz to 1 kHz. The range is further preferred from 5 Hz to 500 Hz, from 10 Hz to 250 Hz and 50 Hz or 200 Hz and most preferably from 20 to 120 Hz. At these frequencies the felting needle can be moved without tearing single filaments of the material while at the same time providing a sufficient amplitude. At the same time, the feltable material can be attached quickly to the target site.

In a preferred embodiment the drive assembly is adapted to drive the reciprocal motion with an amplitude of 2 to 25 mm, particularly preferred 5 to 20 mm, further preferred from 6 to 16 mm most preferred from 8 to 12 mm. These amplitudes cover a sufficient thickness of feltable textile while penetrating the target soft tissue at the same time and result in a sufficiently strong attachment between the feltable material and the target site.

In a preferred embodiment, the fixation device additionally comprises the feltable textile. The feltable textile is arranged or arrangeable such that the at least one needle penetrates the feltable textile with the reciprocal motion. Further preferred, the at least one needle is arranged to penetrate through the feltable textile and into the target site. Thereby, the fixation device and the feltable textile are provided as a unit and are ready to be used together at the target site. As a result, only a single minimally invasive device or at least fewer minimally invasive device need to be inserted into the body of a human or animal.

In a preferred embodiment the at least one felting needle points in a distal direction of the elongate tubular member. This arrangement allows access to target sites that are arranged proximally to a distal tip of the elongate tubular member. In addition, this may allow pressing the feltable textile to the target site, which improves the felting. Further, the needle may be compact in the radial direction and a simple actuation mechanism can be provided.

In a preferred embodiment, the reciprocal motion of the at least one felting needle is linear, particularly preferred along a direction of the elongate tubular member. In an alternative embodiment, the reciprocal motion is rotational, preferably around an axis of the elongate tubular member. The type of reciprocal motion may be chosen depending on the feltable textile and the location of the target site. In case the feltable textile is expelled at the distal end of the elongate tubular member and fixated at the target site distally or proximally thereof, a linear motion is preferable. If the feltable textile is fixated to e.g. a vessel arranged at a radial direction of the elongate tubular member, a rotational motion may be preferable. For example, a feltable textile for an endograft for treating an aneurysm may be preferably fixated with the rotational motion. An annuloplasty ring or a heart valve may be preferably attached with a linear motion.

In a preferred embodiment, the drive assembly comprises an elongate driving wire or tube that extends from a proximal side of the elongate tubular member to the needle assembly. The elongate driving wire or tube is operably connected to the needle assembly such that the driving elongate wire or tube causes the reciprocal motion of the at least one felting needle. Thereby push and/or pull forces may be transmitted from a proximal end of the elongate tubular member to the distal end of the elongate tubular member.

The elongate tubular member as mentioned herein may be described herein as a catheter or sheath (i.e. an outer or inner catheter or sheath).

In a preferred embodiment the drive assembly comprises a resilient element that is arranged such that the resilient element forces the at least one felting needle to a preset position when the at least one felting needle is actuated by the driving elongate wire or tube. Thereby, the reciprocal motion is transmitted onto the at least one felting needle. Alternatively, the driving elongate wire or tube transmits both push and pull forces. In this case, the driving elongate wire or tube may be sufficiently stiff such that compressive forces are transmitted.

In a preferred embodiment the elongate driving wire or tube is configured to transfer tensile and optionally compressive force along the elongate direction of the elongate driving wire or tube to the needle assembly such that the wire or to actuates the reciprocal motion of the at least one felting needle. In a preferred embodiment the wire or tube is made of steel and may form a steel cable.

The driving elongate wire or tube may include a low friction coating on an outer surface. For example, the coating may be of polytetrafluoroethylene (PTFE). Thereby, forces along the longitudinal direction (i.e. compressive or tensile forces) are transmitted efficiently.

In a preferred embodiment, the elongate tubular member comprises at least one guiding element for the drive assembly in the interior lumen. The at least one guiding element preferably centers the elongate driving wire or tube in the elongate tubular member along the radial direction of the elongate tubular member. Thereby, a buckling of the elongate driving wire or tube is reduced, thus improving the efficiency of transmission of forces along the longitudinal direction. Further, vibration in the elongate tubular member may be reduced during operation of the felting needle(s). The guiding elements may also comprise a low friction coating for reducing friction between the driving elongate wire or tube and the guiding elements. This improves the guidance and force transmission of the driving wires or tubes.

In a preferred embodiment the feltable textile has the shape of a strip or patch. Further preferred, the textile may have an annular shape and/or forms an annuloplasty ring or forms part of an annuloplasty ring. Felting an annuloplasty ring is particularly advantageous, since reshaping the annulus of a heart requires a particularly strong and durable attachment due to the compression and expansion of the annuluses of the heart valves.

In a preferred embodiment, the fixation device additionally comprises a holding assembly for the feltable textile, wherein the holding assembly comprises at least one holder for securing the textile to the fixation device. The textile may be held in a folded position. Thereby, the textile and/or an implant comprising the textile has a compact shape and can be advanced to the target site. Further, only one minimally invasive device needs to be inserted that includes the felting needle(s) and a holding assembly with the feltable textile.

In a preferred embodiment, the fixation device additionally comprises a tube with an outer sheath with a distal end. The holding assembly is movable relatively to the outer sheath and upon being expelled from the distal end of the outer sheath, the holding assembly may assume an expanded position such that the textile is unfolded. The holding assembly may be made of a shape memory alloy such as nitinol.

A further aspect relates to a set comprising a fixation device as described above and an implant comprising the feltable textile.

A further aspect relates to a set comprising a fixation device as described above and a guidewire. The feltable textile may be attached or attachable to the guidewire.

A further aspect relates to a set comprising a fixation device as described above and a pusher, preferably a pusher rod. The pusher may be adapted to push the feltable textile to a target site. The pusher may include a distal end face and may be arranged arrangeable within the elongate tubular member. The feltable textile may be arranged a distal end face of the pusher.

A further aspect relates to a method for fixating a feltable textile at the target site in a patient with a minimally invasive fixation device. The fixation device is preferably a fixation device as described above. The method includes the step of guiding a flexible elongate tubular member in the body of the human or animal to a target site. The flexible elongate tubular member has a proximal end, a distal end and an interior lumen. The interior lumen extends between the proximal end and the distal end. A needle assembly is actuated. The needle assembly comprises at least one felting needle arranged at the distal end of the elongate tubular member. The needle assembly is actuated by moving the at least one felting needle relatively to the elongate tubular member with a drive assembly arranged in the interior lumen of the tubular member. The drive assembly is operably connected to the needle assembly and moves the at least one felting needle with a reciprocal motion such that the feltable textile can be fixated at the target site.

The fixation devices, sets and methods as provided herein may be used in the repair and fixation of anatomical structures of the circulatory system (heart and blood vessels), fixation of an implant to these structures, the creation of artificial moderator bands (trabecula septomarginalis) to avoid or treat heart failure with dilated ventricles or cleft closures. In particular, heart defects (e.g. patent foramen ovale, atrial septal defect, ventricular septal defect, patent ductus arteriosus, paravalvular leak closure), heart valve repair and replacements (e.g. aortic valve replacement, mitral valve repair or replacement, tricuspid valve repair or replacement, annuloplasty, chordoplasty), edge-to-edge repair in heart valves and portions such as commissures, repair of blood vessels (e.g. aortic dissections, aneurysms, atherosclerosis), ischemic heart disease (cardio vascular diseases closure, reinforcement of ischemic ventricles and other degenerative cardio vascular diseases may be treated.

In a further aspect, it is suggested to provide a minimally invasive fixation device for fixating a feltable textile at a target site as described above. An end of the at least one felting needle may be directable or directed in a generally distal direction. A generally distal direction may be understood as having a component in the distal direction. Preferably an angle between the distal direction and the end of the felting needle is less than 60°, less than 45 ° or less than 30° or the end of the felting needle points directly in the distal direction. Hereby the user can control the pressure on the tissue and the textile by pulling the fixation device instead of pushing the fixation device. This is particularly of interest where the target site (e.g. area in which surgical felting is performed) is behind anatomical structures such as e.g. valves. In particular, the needle may be curved or angled such that the at least one felting needle points in the generally distal direction.

The fixation device may additionally comprise a feltable textile and the at least one felting needle may be adapted to penetrate the feltable textile from a distal direction. The fixation device may comprise a holding assembly for an implant for the target site and the holding assembly may be adapted to apply a force on the implant in a distal direction. In a distal direction, higher forces can be applied, in particular when the elongate tubular member is bent.

The fixation device may additionally comprise an implant, in particular an annuloplasty ring or a heart valve, wherein the feltable textile is arranged on the implant.

The at least one felting needle may include a deformable joint, in particular a shape memory alloy, such that the at least one felting needle is directed in a generally distal direction when expelled from the interior lumen of the elongate tubular member.

In a further aspect, it is suggested to provide a minimally invasive fixation device for fixating a feltable textile at a target site as described above. The drive assembly may be adapted to move the at least one felting needle in a direction substantially orthogonal to the elongate tubular member with the reciprocal motion.

The at least one felting needle is preferably arranged to rotate around an axis during the reciprocal motion. The axis may be an axis, preferably a central or eccentrical axis, of the elongate tubular member. Thereby, an efficient transmission of the reciprocal motion to the at least one felting needle is provided.

In a preferred embodiment, the driving assembly comprises a driving rod operably connected to the at least one felting needle. The driving rod may be configured to transfer torque onto the needle assembly for rotating the at least one felting needle with the reciprocal motion.

In a preferred embodiment, the driving rod and/or the at least one felting needle is positioned as an eccentric position in the inner lumen of the elongate tubular member. Thereby, the at least one felting needle can be advanced to the inner lumen of the elongate tubular member.

In a preferred embodiment the at least one felting needle is directed or correctable in a direction substantially orthogonal to the elongate tubular member. In this way tissue laying in a radio position relatively to the catheter can be reached. For example, the feltable textile can be attached to walls of blood vessels through which the elongate tubular member may be advanced.

In a preferred embodiment, the at least one felting needle is curved or angled such that a distal tip of the needle is directed in the direction substantially orthogonal to the elongate tubular member. The at least one felting needle may have a curvature of a circle.

In a further aspect, it is suggested to provide a minimally invasive fixation device for fixating a feltable textile at a target site as described above. The fixation device may comprise a tubular outer sheath with a distal end, wherein the elongate tubular member is arranged in the outer sheath and wherein the elongate tubular member can be bent, in particular when the elongate tubular member is expelled from the distal end of the outer sheath such that the distal end of the elongate tubular member is steerable by rotating the elongate tubular member. Thereby, the needle assembly can be navigated to the feltable textile. In particular, the needle assembly can be rotated such that the feltable textile can be fixated along a circular track.

The elongate tubular member may comprise low friction outer coating such as PTFE or be made of a low friction material. Alternatively or additionally, the outer sheath may comprise a low friction outer coating (e.g. PTFE) or be made of a low friction material. As used herein any coating that reduces friction as compared to the base material may be considered to be a low friction coating. Material combinations which result in a dynamic coefficient of friction below 0.1 may be considered as "*low friction*". (e.g. Teflon (PTFE) on teflon, or teflon on steel is around 0.04).

In a preferred embodiment, the elongate tubular member comprises a joint between its distal and proximal ends, wherein the elongate tubular member can be bent at the joint. Particularly preferred, the joint comprises a shape memory alloy. The joint may assume a bent position upon being expelled from the outer sheath.

Additionally, the elongate tubular member may comprise wires or cables or a pneumatic or hydraulic mechanism for rotating the elongate tubular member around its axis.

In a further aspect, it is suggested to provide a minimally invasive fixation device for fixating a feltable textile at a target site as described above. The elongate tubular member may comprise a distal tip portion that is arranged at least partially distal of the at least one felting needle, wherein the distal tip portion comprises a proximal counter surface opposed to the at least one felting needle for holding the feltable textile in place during fixation. Thereby, the tissue of the human or animal body may be held, e.g. clamped, in place during the felting. In alternative embodiments, the distal tip portion may also be arranged proximally of the at least one felting needle.

In a preferred embodiment, the distal tip portion includes a slot for receiving the feltable textile. The slot may be partially formed by the distal counter surface.

In a preferred embodiment, the distal tip portion may be formed by one, two, three or more tongues that are formed to provide a distal counter surface for the felting.

In a further aspect, it is suggested to provide a minimally invasive fixation device for fixating a feltable textile at a target site as described above. The fixation device may further comprise a textile holder and a feltable textile. At least a part of the feltable textile is held at the distal end of the elongate tubular member with the textile holder such that the feltable textile covers the needle assembly at least partially. The at least one felting textile is configured to penetrate the feltable textile.

In a preferred embodiment, the feltable textile has an elongate shape, a first end, an intermediate portion and a second end along the elongate extension. The elongate tubular member includes at least one, preferably two, guidance openings, preferably slots, for holding the feltable textile such that the feltable textile covers the new assembly at least partially. Thereby, the feltable textile may be hold in place during delivery and during fixation.

In a preferred embodiment the textile holder comprises a clamp for a frictional engagement between the feltable textile and the textile holder. Further preferred, the catheter assembly comprises a textile holder release mechanism for releasing the textile from the fixation device, wherein the textile holder release mechanism preferably comprises a textile with cables. Thereby, the feltable textile may be released from the fixation device. In a further preferred embodiment, the catheter assembly comprises a cutting-edge, preferably a knife for cutting the feltable textile. The cutting edge maybe rotatable, which allows an easy actuation of the textile cutting mechanism.

Nonlimiting embodiments of the invention are described, by way of example only, with respect to accompanying drawings. The drawings and its description provide examples of the invention, individual features of which may be used alone or in combination with each other.
Figure 1A: is a schematic view of an end portion of a first fixation device in a first stage of fixation.
Figure 1B: is a schematic view of an end portion of the fixation device of figure 1A in a second stage of fixation.
Figure 2A: is a schematic view of an end portion of the fixation device of figure 1A in a third stage of fixation.
Figure 2B: is a schematic view of an end portion of the fixation device of figure 1A in a fourth stage of fixation.
Figure 3: shows a schematic view of a cross-section of a second fixation device
Figure 4A to 4C: show a schematic view of the third fixation device with a textile patch and an outer sheath.
Figure 5A to 5C: show a schematic view of the third fixation device with a textile patch in the form of an annuloplasty ring and an outer sheath.
Figure 6: shows a schematic view of the third fixation device with the textile patch after implantation.
Figure 7: is a schematic view of an end portion of a fourth fixation device.
Figures 8A to 8B: are schematic view of the fourth fixation device during fixation.
Figures 9A to 9C: are cross-sectional views of a portion of fixation device showing example embodiments of needle assemblies.
Figures 10A to 10B: show a schematic view and a cross-section of a fifth fixation device.
Figures 11A to 11D: are schematic views of an end portion of a sixth fixation device in four stages of fixation.
Figure 12: is a schematic view of a cross-section of a human heart after fixation of a of an end portion of the fixation device of figure 1 in a fourth stage of fixation.

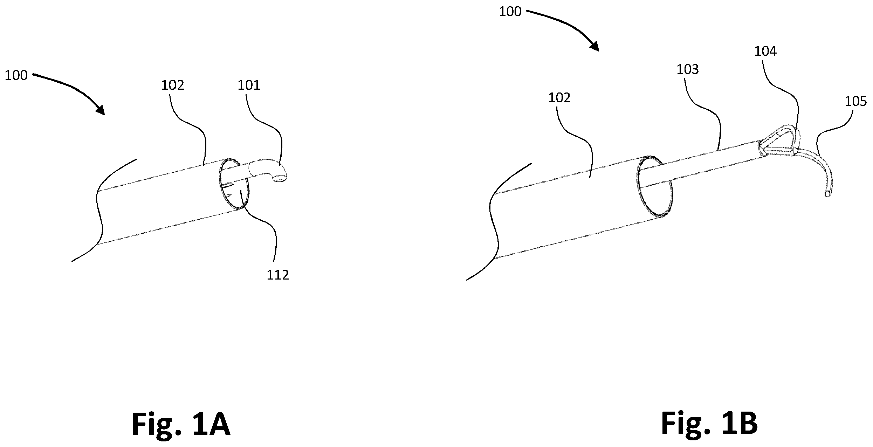

Figures 1A to 2B show an end portion of the first embodiment 100 of a fixation device. The fixation device 100 includes an elongate tubular member that is realized as a catheter 102. The catheter 102 may be inserted into the body of a human or animal, for example through a short sheath with a valve (i.e. a portal). One example of the insertion of such a short sheath to gain access to a blood vessel and other hollow organs is the Seldinger technique. Before the catheter 102 is inserted, a guidewire 101 is inserted. The guidewire 101 navigates the vessels to reach a target site, such as particular vessel segments, e.g. heart valves or lesions. Then, the catheter 102 is pushed over the guidewire 101 to the target site.

The situation, where the catheter 102 has reached the target site is shown in figure 1A. Thereafter, hooks 104 and 105 are deployed (see figure 1B). The hooks 104, and 105 may include a sharp tip to anchor the device in soft tissue to retain a location of the fixation device at the target site. The hooks 104, 105 may penetrate the tissue or may reach behind the tissue and pull the tissue towards the catheter 102. Alternatively, the guidewire 101 is retracted after the catheter 102 has reached the target site, and a second catheter 103 is introduced which includes the hooks 104, and 105.

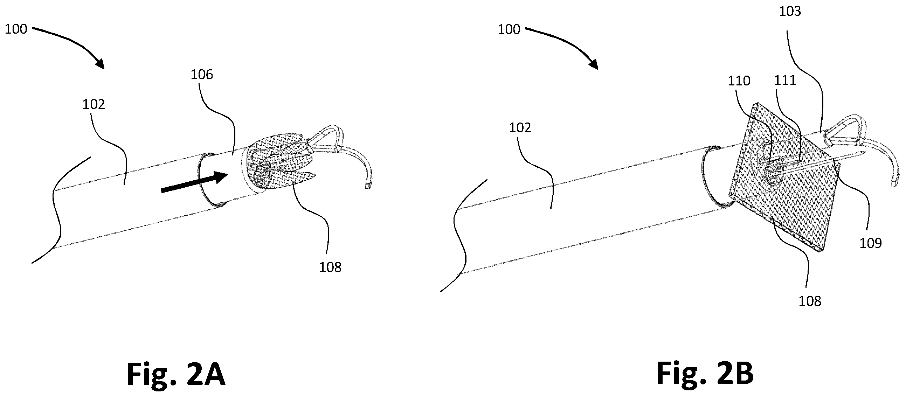

In the next step, a pusher 106 is advanced through the catheter 102 over the guidewire 101 or over the second catheter 103. On its distal end, the pusher 106 includes a flat surface. On the flat surface, a folded patch 108 made of a feltable textile is arranged and pushed through an inner lumen of the catheter 102. When the folded patch is expelled from a distal end of the catheter 102 as shown in figure 2A, it unfolds as shown in figure 2B. The flat side of the distal end of the pusher 106 is used to push the patch 108 against the tissue of the human or animal. In order to prevent the patch 108 from moving, the pusher includes two barbed needles 110, 111 that are deployed, i.e. moved in a distal direction through the patch and into the tissue of the human or animal to keep the patch in place. Then, a felting needle 109 is moved through the patch 108 and into the soft tissue of the human or animal, Then, the felting needle 109 is retracted again by moving the needle in the proximal direction. The advancement and retraction are repeated with a frequency of 60 Hz. By repeatedly advancing a needle 109 through the patch 108 the fibers of the patch 108 are pushed or pulled through patch 108 into the soft tissue 3 by means of the needle 109 to produce a firm connection between the feltable textile of the patch and the soft tissue. This process may be supported by the needle additionally comprising felting barbs as described in PCT/CH2019/000015. Thereafter, the patch 108 is fixated at the target site.

The needle can be moved over the surface of patch 108 by rotating the catheter 102 around the second catheter 103. To cover a greater area this can be repeated by navigating to a new target site on the patch with catheter 102.

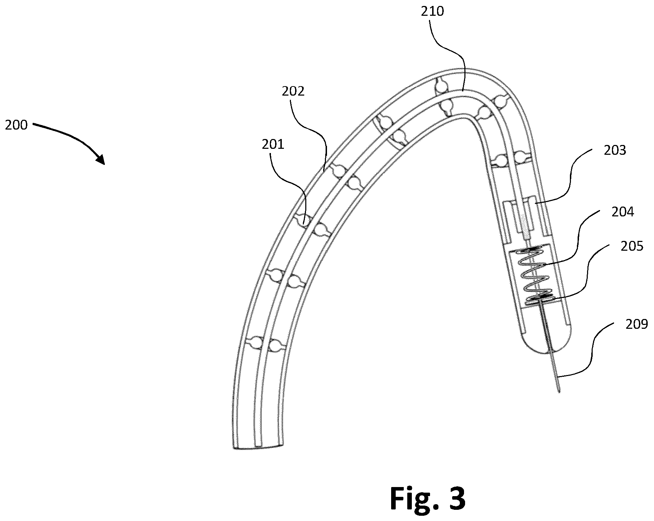

Figure 3 shows a schematic view of a cross-section of a second fixation device. Figure 3 illustrates how a felting needle, for example felting needle 109, may be actuated. Patches of feltable materials such as patch 108 may be attached to target sites in the cardiovascular, urological, gastrointestinal, or neurovascular regions. Oftentimes this requires following the path of the respective hollow organ, e.g. blood vessels, along their bent paths. Therefore, catheters that are flexible, and sometimes soft, are used to allow the catheter to follow the shape of the hollow organ. However, this poses a challenge for actuating the felting needle with the reciprocal motion. In particular in case the fixation device comprises a motor to actuate the felting needle, the motion needs to be transmitted to the felting needle via a driving assembly. One example of such a driving assembly is shown with reference to figure 3. Figure 3 shows a fixation device 200 with a felting needle 209. An actuator (not shown) generates the reciprocal motion and is directly connected to a driving wire 210. The driving wire 210 is pulled by the motor in the proximal direction. Similarly to the fixation device 100, the fixation device 200 comprises an elongate tubular member that is realized as a catheter 202. As can be seen from figure 3, the catheter 202 is flexible along its entire length and bends around curves. The driving wire 210 is held and guided within the catheter 202 by a guiding element. The guiding element may be a concentric lead element 201 as shown in figure 3. The concentric lead elements 201 are circular, ring-shaped and extend from an inner side of the catheter. Those elements 201 have preferably also a low friction index to allow optimal guidance and force transmission. This feature reduces bending forces and ultimately reduces vibration during application. The concentric lead elements 201 center the driving wire in the catheter 202, thereby preventing a bending or buckling of the driving wire in the tube beyond the bending of the catheter 202. The concentric lead elements are covered by a PTFE coating to lower a friction between the driving wire 210 and the lead elements. Additionally or alternatively, the driving wire may also include a similar or different friction reducing (low friction) coating.

The catheter 202 comprises a linear bearing 203 at its distal end. Further, the catheter 202 includes a spring coil 204 and a felting needle 209 at its distal end. The driving wire 210 is rigidly connected to the felting needle 209 or forms the felting needle 209 and is connected to a distal end of the spring coil 204 with a washer 205. On the other hand, the spring coil contacts the linear bearing 203. When the wire 210 is pulled, the needle 209 is retracted and the spring 204 is compressed between the linear bearing 203 and the washer 205. Upon releasing the wire 210, the spring coil presses the felting needle forward. The amplitude (penetration depth) and frequency of the motion of the felting needle can be modulated and the suitable springs with resonance frequencies in the desired range may be chosen as is known in the art.

If a sufficiently stiff driving wire is used (e.g. steel cable) which is able to transport compressive and tensile forces from one end to the other end of the catheter, the spring 204 can be avoided. Alternatively to the above described driving assembly comprising an elastic element (spring 204) and a driving wire, the needling mechanism can be driven by a pneumatic or hydraulic force or by an electro-magnetic actuator.

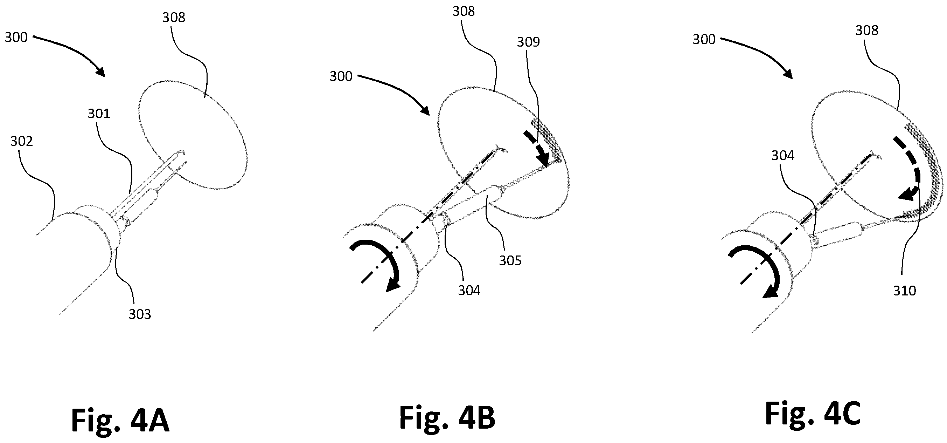

Figures 4A to 4C show a schematic view of the third fixation device 300 with a textile patch 308 and an outer sheath 302. In particular, figures 4A to 4C show one embodiment of how a patch of a feltable textile 308 may attached to a target site of soft tissue. In the embodiment of figures 4A to 4C the feltable textile 308 is advanced with a guidewire 301. In first step of the operation the guidewire 301 is guided towards the target site as is known in the art. The tip of the guidewire is for example angled e.g. by having a J shape, allowing a navigation through hollow organs, such as blood vessels. Once, the guidewire 301 is at the target site, an outer sheath 302 is advanced over the guidewire into the body of the human or animal. The outer sheath 302 comprises an inner lumen, through which a further outer catheter 303 is advanced. The outer catheter 303 includes an inner catheter 305. The inner catheter 305 may be a catheter such as catheter 202 as described with reference to figure 3. The patch 308 can be provided with a small hole or simply be penetrated by the guidewire. Then the patch can be advanced over the guidewire 301 with a separate pusher (not shown). Otherwise the patch 308 can be advanced with the guidewire 301 when the guidewire is inserted or by being pushed with the outer sheath 302 or with the outer or inner catheter 303, 305 over the guidewire 301.

In addition, the inner catheter 305 comprises a joint 304. The joint may comprise an elastic element such as a coil or an elastic material and a bearing, e.g. a pivot bearing. The elastic element is prestressed and forces the inner catheter to bend at the joint 304 around the bearing. For example, the elastic material may be compressed which would bend the inner catheter to an opposing side or the elastic material may be stretched, which would bend the inner catheter to the side of the elastic material. The angle of the joint may in one example be controlled by a user with a wire pulling the elastic element.

Additionally or alternatively, the joint 304 is made of a shape memory alloy. Further, upon being expelled from the outer catheter 303, the joint bends the inner catheter 305 at a predetermined angle. The angle may be controlled by the axial position of the inner catheter 305 relative to the outer catheter. This allows the needle to cover a bigger felting area on the patch.

The predetermined angle may be less than 90°, preferably less than 60°, preferably less than 45° and particularly preferred about 30°. Due to the bending at the joint, a rotation of the inner catheter 305 around its own axis steers the distal tip of the inner catheter 305 similarly to a guidewire with a J-shaped end. Thereby, the end portion of the inner catheter with the felting needle can be guided to a desired position. As shown in particular in figures 4B and 4C (see arrows 309, 310), the steerable inner catheter is rotated around its own axis and thereby the circumference of the patch 308 is attached to the target site with the felting needle. The relative position of the inner catheter 305 and the outer catheter 303 may be adjustable. For example, at a first relative position, the joint may be end at an angle of 15° at a first relative position while the angle may be 30° at a second position. Thereby, an inner radius can be fixated. For example, the further the outer catheter 303 is withdrawn, the further the inner catheter 305 bent. Thereby, the entire surface of the eligible material can be attached to the target site by rotating the inner catheter and changing the angle of the joint 304.

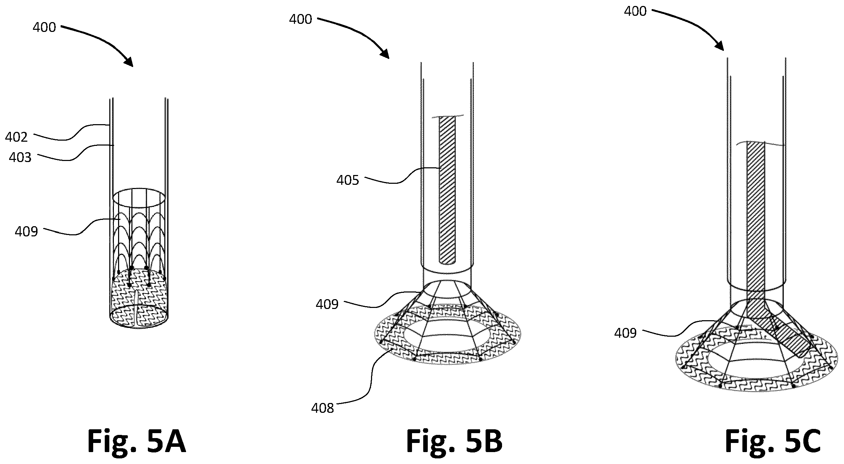

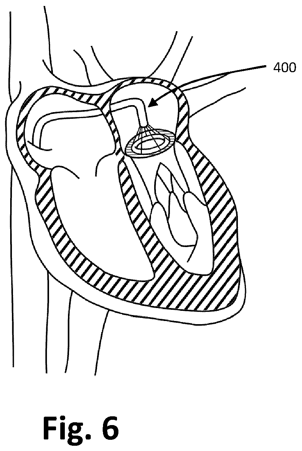

A particular application of the above technique is shown with respect to figures 5A to 6. In this application, the feltable textile forms an annuloplasty ring 408. An annuloplasty is a procedure to tighten or reinforce the ring (annulus) around a valve in the heart. The annuloplasty ring reshapes, reinforces and/or tightens the ring around a heart valve thereby preventing for example regurgitation through the valve. An outer catheter 403 includes a holder 409 for the feltable textile 408 that is ring-shaped. The holder has a mesh structure and is made of a shape memory alloy that expands upon being expelled from an outer sheath 402. The holder 409 includes releasable anchors that may clamp the feltable textile. When the holder is expelled from the distal end, it spans the feltable textile 408. Then, similarly to the inner catheter 305 of figures 4A to 4C, an inner catheter 405 is advanced through the outer catheter 403. Similarly to the inner catheter 305 of figures 4A to 4C, the inner catheter 405 includes a bendable joint. Through a rotation around the axis of the inner catheter, the annuloplasty ring 408 is attached to an annulus of a heart valve. The annuloplasty ring is pressed against the tissue by pushing the catheter 403 in the proximal direction. The result of the procedure is shown in figure 6. Here the annuloplasty ring 408 is attached to the mitral valve of a human heart on the side of the left atrium.

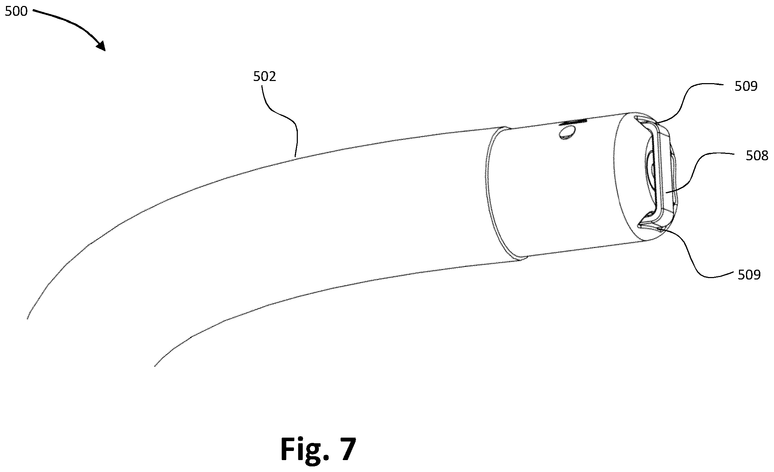

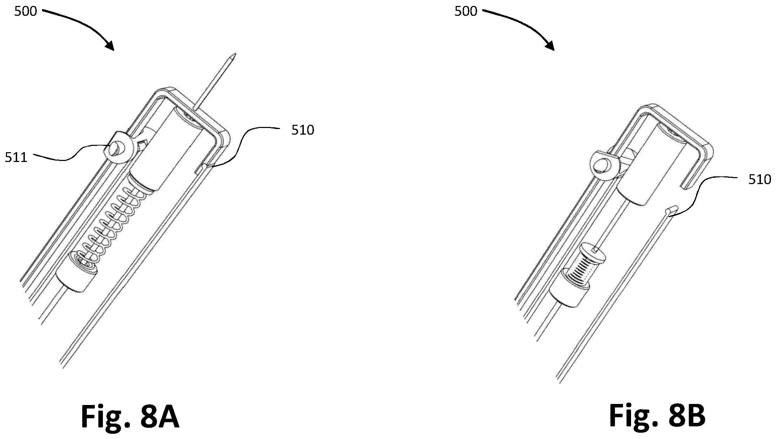

A further embodiment 500 of a fixation device is shown in figures 7 to 8B. The fixation device 500 includes an elongate tubular member formed as catheter 502. In this fixation device, the eligible textile is provided as an elongate feltable textile strip 508 (e.g. an elongate patch or a braided suture). The feltable textile strip 508 extends along the surface of the tip of the catheter 502 and to goes into two slots 509 provided at the tip of the catheter 502. Figures 8A and 8B show a view, in which the catheter 502 is removed. As can be seen from figures 8A and 8B, the fixation device includes similar as elements as the catheter of figure 3. In particular, the device comprises a spring coil, a linear bearing, a driving wire, a washer and a felting needle that can be advanced and retracted with the driving wire.

In addition, the device comprises two holders 510, 511 for the strip 508. A first holder 510 clamps an end of the strip 508. The second holder 511 also clamps the strip 508. On the side of the second holder, the strip 508 extends further along the length of the catheter 502 in the proximal direction. In some embodiments the strip may go along half or the entire catheter from the catheter tip. The catheter 502 also provides an inner lumen for a guidewire and a steering wire for positioning.

Strip 508 enters the catheter on one side and is stretched by the felt holders 510, 511 to the opposite side, where it is attached. In another embodiment a felt holder only holds the textile over the tissue wall and a fixation device as previously described with reference to figure 3 starts fixating it. The holder 511 can release the strip after it is felted to a soft tissue by being rotated (for example with a release cable), and the catheter tip can be moved along a surface to which the strip 508 is to be attached. The catheter tip can be moved along a desired route, which pulls out the strip 508 continuously. Once the strip is felted to the soft tissue over a desired length, the felt strip is cut by rotating the holder 511 further. The holder 511 includes a cutting edge. By rotating the holder 511 further, the strip is cut. Then, or beforehand the first holder is released as well. Thereby, a strip of feltable textile is applied similarly to a correction roller. The feltable textile strip can be advanced for example by manual or electrical means.

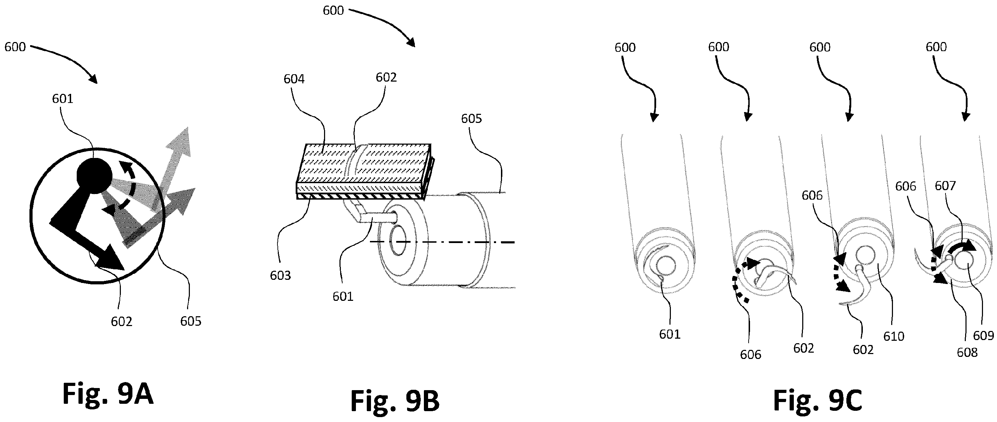

In the previous embodiments, the felting needle was moved linearly along the elongate direction of the respective catheters. However, the needle may also rotate or move in a direction orthogonal to the catheter. An example of such a needle is shown with respect to figures 9A to 9C. Figure 9A shows a cross-section of a catheter 605 of a fixation device 600. In the cross-section, a felting needle 602 that is connected with a rod 601 is shown. In the position shown in figure 9A and on the left side of figure 9C, the needle may be advanced through the catheter 605. As can be seen from figures 9A to 9C, the needle may either be angled or curved, in particular curved along an arc, e.g. a circular circumference. Further as can also be seen from figures 9A to 9C, the rod 601 is arranged eccentrically in the catheter 605. Once the needle exits a distal end of the catheter 605, the rod 601 may be rotated around its axis as indicated by arrow 606. Then, the needle 602 can be used to felt a strip of felt of the material 603 to a tissue 604 as shown in figure 9B. Since the rod 601 and thus the needle 602 are arranged eccentrically, tissue 604 and feltable textile 603 can be attached to each other. Similarly to the previous embodiments, the needle is moved back and forth with a reciprocal motion to felt tissue 604 and textile 603. In this way, tissue laying in radial position relative to the catheter can be reached, e.g. in blood vessels.

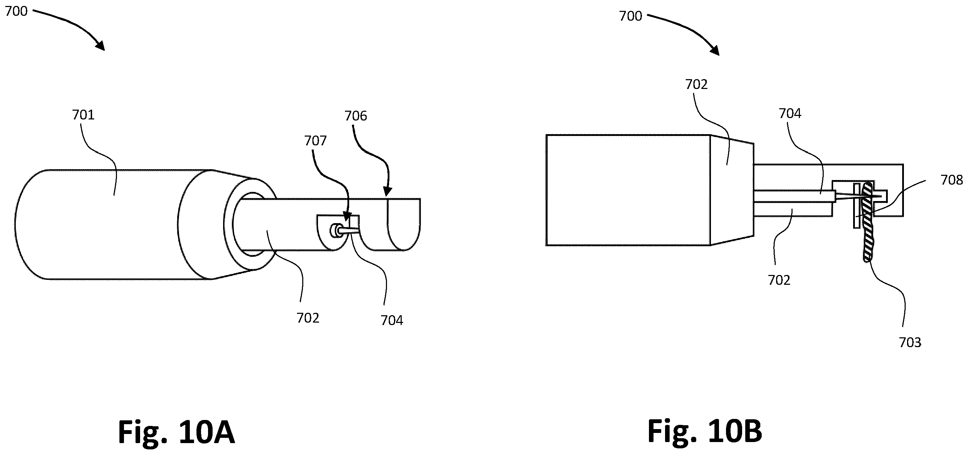

Additionally, the eccentric position of the rod 601 may be changed by moving the rod 601 relatively to the catheter to another eccentric position. For example, as shown to the right in figure 10C, the rod 601 is movable along a circular path within the cross-section of the catheter 605 as indicated by arrow 607.The rod 601 can be moved to different circumferential positions of the catheter 605. The positions may be defined by an inner wall, e.g. inner catheter 608, and a core 609. The core 609 may be a tube for the guidewire and is arranged within the lumen of the inner catheter. Thereby, a space 610 is formed between the inner catheter 608 and the core 609 that extends along the length of the inner catheter 608 and the core 609. In the example of figure 10C the space has the shape of a hollow cylinder.

Figures 10A to 10B show a schematic view and a cross-section of a fifth fixation device. The fifth fixation device 700 includes an outer sheath 701 and a catheter 702. The catheter 702 additionally comprises a distal tip portion 706 with a slot 707 for receiving a feltable textile 708 and tissue 703. On an opposing side of a felting needle 704, a distal counter surface of the slot 707 is arranged. The distal tip portion and the counter surface hold the feltable textile and the tissue in place during fixation by generating resistance and keeping feltable textile 708 and tissue 703 in place.

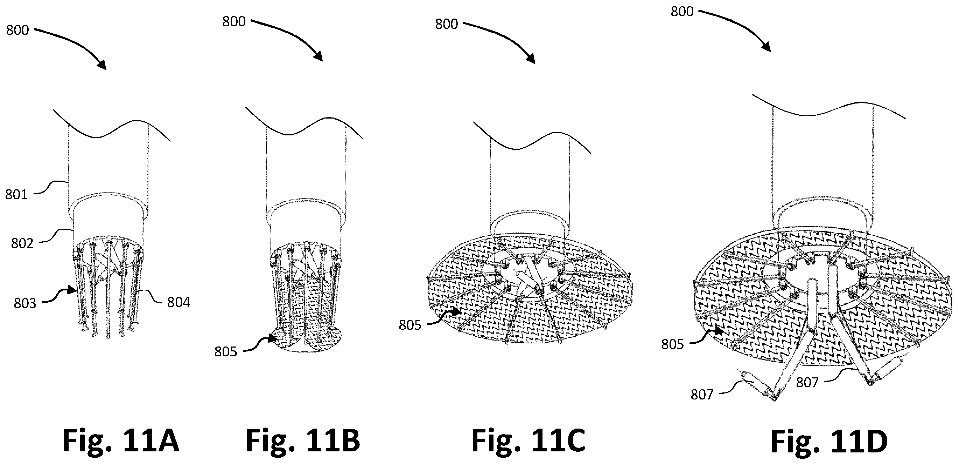

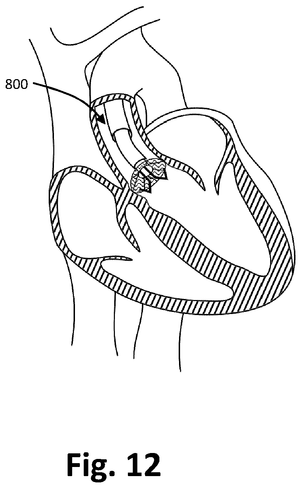

A further embodiment of a fixation device 800 is shown with reference to figures 11A to 12. Figures 11A to 11B show the fixation device 800 in isolation while figure 12 illustrates an example implantation of the fixation device 800 in the annulus of the aortic valve of a human. The fixation device 800 includes an outer sheath 801 and an inner sheath 802. A holder 803 for an implant comprising a feltable textile is arranged at the distal end portion of the inner sheath 802. The holder includes a plurality of arms 804 as can be seen in figure 11A. These arms 804 hold the feltable textile that forms an annuloplasty ring 805 for the aortic valve in the shown embodiment as can be seen from figures 11B to 12. The arms 804 may clamp, grasp, be laced through the feltable textile or otherwise fixate the annuloplasty ring to hold the annuloplasty ring. Similarly to the holder 409, the arms 804 may be made of a shape memory alloy or comprise one or more joints made of a shape memory alloy. Thus, when the arms are expelled from the outer sheath 801, they fold out radially and bring the annuloplasty ring in a position in which the annuloplasty ring 805 can be attached to a soft tissue.

The embodiment shown in figures 11A to 12D additionally includes two felting needles 806 with a driving assembly comprising two arms 807. The driving assembly is moved past the annuloplasty ring (i.e. through the hole of the ring) in the distal direction. Upon being expelled from the inner sheath 802, the arms unfold or are unfolded, such that the felting needles at their end point in a distal direction. Then, the two felting needles are actuated such that the felting needles stitch the annuloplasty ring proximally from a distal direction and felt the annuloplasty ring to a soft tissue proximally behind the annuloplasty ring. The shown arrangement allows a user to control the pressure on the tissue by pulling at the inner sheath 802. This is particularly of interest where the target zone (area in which surgical felting is performed) is behind anatomical structures such as e.g. valves. Alternatively, the needles themselves might be curved or angled such that their tips point in a distal direction.

One application of the above described fixation devices and feltable textile is a mitral valve repair, optionally with replacement/enhancement of the chordae tendineae. During diastole, the opening of the mitral valve lets blood flow from the left atrium to the left ventricle. During systole, the mitral valve closes, so that blood gets pumped through the aorta to the body. Patients suffering from mitral regurgitation have a mitral valve that does not close tightly, which allows blood to flow from the left ventricle back to the left atrium. Depending on cause and severity of the case, one approach is to replace or enhance the chordae tendineae; the cords that transmit the contraction and relaxation of the papillary muscles to the mitral valve. Access to the mitral valve may be gained percutaneously through the femoral vein and puncture of the septum. The catheter enters the mitral valve and fixates the first end of a textile patch to the papillary muscle. A second end is fixated at the leaflet and gets released by the catheter. Thereby, the chordae tendineae are supported by the feltable textile that are fixated strongly and durably. In particular, the device shown with respect to figures 7 to 8B might be used for this purpose.

Another application of the above described fixation devices and feltable textiles is an Atrial Septal Defect (ASD). ASD is a congenital heart defect that occurs, when the septum between the two atria is not developed properly and a hole remains. Depending on the size and location of the defect, the hole needs to be closed. The majority of cases can be treated by a transcatheter approach shown above. Access to the defect of the septum is gained percutaneously through the femoral vein into the right atrium. The elongate tubular member in the form of a catheter contains a central rod with a folded disk at the tip, which is guided through the hole into the left atrium. There, the feltable textile can be unfolded and expanded. A holder as described above may fixate the feltable textile. The pusher may also push the folded textile that will occlude the hole. It gets pushed through the catheter, unfolds once it exits the catheter and is pressed onto the septum. Two hollow tubes, each equipped with a barbed needle next to the central rod are guided to the patch. They fixate the edge of the patch by rotating (in this case 90°) in both directions (clockwise and counterclockwise) and thereby occluding the hole as shown in figures 4A to 4C. Alternatively, this surgery could also be performed with a continuous deployed feltable textile as shown with respect to figures 7 to 8B.

In a further example of a surgical procedure, the feltable textile is advanced through the septal defect, such that the main section is positioned in the septal defect, the first connecting section is arranged in the left atrium and the second connecting section is arranged in the right atrium, or vice versa. An upper portion of the first connecting section may then be connected to the upper part of the atrial septum adjacent the atrial septal defect, and an upper portion of the second connecting section may be connected to the upper part of the atrial septum by advancing a surgical felting needle repeatedly through the respective portion of the respective connecting section and into the atrial septum. **In** this manner, the fibers of the respective connecting section are inserted into the atrial septum to tightly connect the medical implant to the heart tissue. Subsequently, a mechanical force (e.g. an upward force on the lower part of the heart and hence the lower part of the atrial septum or a downward force on the upper part of the heart and hence the upper part of the atrial septum) may be applied to transiently close the septal defect, and the lower portion of the first connecting section and the lower portion of the second connecting section may then be connected to the heart tissue of the lower part of the atrial septum by advancing a surgical felting needle repeatedly through the respective portion of the respective connecting section and into the tissue of the lower part of the atrial septum. The described procedure is preferably performed using minimal invasive surgery, i.e. by advancing one or several catheters into the left atrium and or the right atrium of the heart via the vascular system of the patient.

## Claims

1. A minimally invasive fixation device (100; 200; 300; 400; 500; 600; 700; 800) for fixating a feltable textile (108; 308; 408; 508) at a target site in a human or animal comprising:
- an elongate tubular member (202) having a proximal end and a distal end, an interior lumen extending between the proximal end and the distal end, wherein the elongate tubular member is at least partially flexible,
- a needle assembly comprising at least one felting needle (209) arranged or arrangeable at the distal end of the elongate tubular member, wherein the at least one felting needle is movable relatively to the elongate tubular member, and wherein the at least one felting needle is movable with a reciprocal motion, and
- a drive assembly (204, 205, 210) arranged in the interior lumen of the tubular member, wherein the drive assembly is operably connected to the needle assembly for moving the at least one felting needle with the reciprocal motion when the at least one felting needle is located at the distal end such that the feltable textile can be fixated at the target site.

2. Fixation device according to claim 1, wherein the drive assembly is adapted to drive the reciprocal motion with an amplitude of in the range of 2 to 25mm, preferred from 6 to 16mm most preferred from 8 to 12mm.

3. Fixation device according to one of the previous claims, wherein the fixation device additionally comprises the feltable textile (108; 308; 408; 508) and wherein the feltable textile (108; 308; 408; 508) is arranged or arrangeable such that at least one felting needle (209) penetrates the feltable textile with the reciprocal motion.

4. Fixation device according to one of the previous claims, wherein the at least one felting needle (209) points in a distal direction of the elongate tubular member.

5. Fixation device according to one of the previous claims, wherein the reciprocal motion of the at least one felting needle is linear or rotational.

6. Fixation device according to one of the previous claims, wherein the drive assembly comprises an elongate driving wire (210) or tube that extends from a proximal side of the elongate tubular member to the needle assembly, and wherein the elongate driving wire or tube is operably connected to the needle assembly such that the driving elongate wire or tube causes the reciprocal motion of the at least one felting needle.

7. Fixation device according to claim 6, wherein the drive assembly comprises a resilient element (204) that is arranged such that the resilient element forces the at least one felting needle back to a preset position when the at least one felting needle is actuated by the driving elongate wire or tube.

8. Fixation device according to claims 6 or 7, wherein the elongate driving wire (210) or tube is configured to transfer tensile and optionally compressive force along the elongate direction of the elongate driving wire (210) or tube to the needle assembly such that the driving wire (210) actuates the reciprocal motion of the at least one felting needle.

9. Fixation device according to one of claims 6 to 8, wherein the elongate driving wire (210) or tube includes a low friction coating on an outer surface, in particular a polytetrafluorethylene coating.

10. Fixation device according to one of the previous claims, wherein the elongate tubular member comprises at least one guiding element (201) for the drive assembly in the interior lumen.

11. Fixation device according to one of the previous claims, including the feltable textile (308), wherein the feltable textile has the shape of a strip (508) or patch (308).

12. Fixation device according to one of the previous claims, comprising the textile, wherein the textile has an annular shape and/or forms an annuloplasty ring (805).

13. Fixation device according to one of the previous claims, wherein the fixation device additionally comprises a holding assembly for the feltable textile, wherein the holding assembly comprises at least one holder (409; 510; 511) for securing the textile to the holding assembly, wherein the textile is in a folded position.

14. Fixation device according to claim 13, comprising a tubular outer sheath (402) with a distal end, wherein the holding assembly is movable relatively to the outer sheath and wherein, upon being expelled from the distal end of the outer sheath, the holding assembly assumes an expanded position such that the textile is unfolded.

15. A set comprising a fixation device according to one of the previous claims, and an implant comprising the feltable textile.

## Patentansprüche

1. Minimalinvasive Fixiervorrichtung (100; 200; 300; 400; 500; 600; 700; 800) zum Fixieren eines filzbaren Textils (108; 308; 408; 508) an einer Zielstelle in einem Menschen oder Tier, aufweisend:
ein längliches rohrförmiges Element (202) mit einem proximalen Ende und einem distalen Ende, einem sich zwischen dem proximalen Ende und dem distalen Ende erstreckenden inneren Lumen, wobei das längliche rohrförmige Element zumindest teilweise flexibel ist;
eine Nadelanordnung, die mindestens eine Filznadel (209) aufweist, die am distalen Ende des länglichen rohrförmigen Elements angeordnet oder anordenbar ist, wobei die mindestens eine Filznadel relativ zum länglichen rohrförmigen Element bewegbar ist, und wobei die mindestens eine Filznadel mit einer hin- und hergehenden Bewegung bewegbar ist; und
eine Antriebsanordnung (204, 205, 210), die im inneren Lumen des rohrförmigen Elements angeordnet ist, wobei die Antriebsanordnung mit der Nadelanordnung betrieblich verbunden ist, um die mindestens eine Filznadel mit der hin- und hergehenden Bewegung zu bewegen, wenn die mindestens eine Filznadel am distalen Ende angeordnet ist, so dass das filzbare Textil an der Zielstelle fixierbar ist.

2. Fixiervorrichtung nach Anspruch 1, wobei die Antriebsanordnung dazu eingerichtet ist, die hin- und hergehende Bewegung mit einer Amplitude im Bereich von 2 bis 25 mm, vorzugsweise von 6 bis 16 mm, am bevorzugtesten von 8 bis 12 mm, anzutreiben.

3. Fixiervorrichtung nach einem der vorangehenden Ansprüche, wobei die Fixiervorrichtung zusätzlich das filzbare Textil (108; 308; 408; 508) aufweist, und wobei das filzbare Textil (108; 308; 408; 508) derart angeordnet oder anordenbar ist, dass mindestens eine Filznadel (209) das filzbare Textil mit der hin- und hergehenden Bewegung durchdringt.

4. Fixiervorrichtung nach einem der vorangehenden Ansprüche, wobei die mindestens eine Filznadel (209) in eine distale Richtung des länglichen rohrförmigen Elements zeigt.

5. Fixiervorrichtung nach einem der vorangehenden Ansprüche, wobei die hin- und hergehende Bewegung der mindestens einen Filznadel linear oder rotierend ist.

6. Fixiervorrichtung nach einem der vorangehenden Ansprüche, wobei die Antriebsanordnung einen länglichen Antriebsdraht (210) oder ein längliches Antriebsrohr aufweist, der bzw. das sich von einer proximalen Seite des länglichen rohrförmigen Elements zur Nadelanordnung erstreckt, und wobei der längliche Antriebsdraht oder das längliche Antriebsrohr betrieblich mit der Nadelanordnung verbunden ist, so dass der längliche Antriebsdraht oder das längliche Antriebsrohr die hin- und hergehende Bewegung der mindestens einen Filznadel bewirkt.

7. Fixiervorrichtung nach Anspruch 6, wobei die Antriebsanordnung ein elastisches Element (204) aufweist, das derart angeordnet ist, dass das elastische Element die mindestens eine Filznadel in eine voreingestellte Position zurück zwingt, wenn die mindestens eine Filznadel durch den länglichen Antriebsdraht oder das längliche Antriebsrohr betätigt wird.

8. Fixiervorrichtung nach Anspruch 6 oder 7, wobei der längliche Antriebsdraht (210) oder das längliche Antriebsrohr dafür konfiguriert ist, eine Zugkraft und optional eine Druckkraft entlang der Längsrichtung des länglichen Antriebsdrahts (210) oder des länglichen Antriebsrohrs auf die Nadelanordnung zu übertragen, so dass der Antriebsdraht (210) die hin- und hergehende Bewegung der mindestens einen Filznadel betätigt.

9. Fixiervorrichtung nach einem der Ansprüche 6 bis 8, wobei der längliche Antriebsdraht (210) oder das längliche Antriebsrohr auf einer Außenfläche eine reibungsarme Beschichtung, insbesondere eine Polytetrafluorethylenbeschichtung, aufweist.

10. Fixiervorrichtung nach einem der vorangehenden Ansprüche, wobei das längliche rohrförmige Element mindestens ein Führungselement (201) für die Antriebsanordnung im inneren Lumen aufweist.

11. Fixiervorrichtung nach einem der vorangehenden Ansprüche, die das filzbare Textil (308) aufweist, wobei das filzbare Textil die Form eines Streifens (508) oder eines Flickens (308) aufweist.

12. Fixiervorrichtung nach einem der vorangehenden Ansprüche, die das Textil aufweist, wobei das Textil eine ringförmige Form aufweist und/oder einen Annuloplastiering (805) bildet.

13. Fixiervorrichtung nach einem der vorangehenden Ansprüche, wobei die Fixiervorrichtung zusätzlich eine Halteanordnung für das filzbare Textil aufweist, wobei die Halteanordnung mindestens einen Halter (409; 510; 511) zum Sichern des Textils an der Halteanordnung aufweist, wobei sich das Textil in einer gefalteten Position befindet.

14. Fixiervorrichtung nach Anspruch 13, aufweisend eine rohrförmige Außenhülle (402) mit einem distalen Ende, wobei die Halteanordnung relativ zur Außenhülle bewegbar ist und wobei die Halteanordnung beim Austreten aus dem distalen Ende der Außenhülle eine expandierte Position einnimmt, so dass das Textil entfaltet wird.

15. Set, aufweisend eine Fixiervorrichtung nach einem der vorangehenden Ansprüche und ein Implantat, das das filzbare Textil aufweist.

## Revendications

1. Dispositif de fixation minimalement invasif (100 ; 200 ; 300 ; 400 ; 500 ; 600 ; 700 ; 800) pour fixer un textile feutrable (108 ; 308 ; 408 ; 508) au niveau d'un site cible chez un humain ou un animal, comprenant :
- un élément tubulaire allongé (202) ayant une extrémité proximale et une extrémité distale, une lumière intérieure s'étendant entre l'extrémité proximale et l'extrémité distale, dans lequel l'élément tubulaire allongé est au moins partiellement flexible,
- un ensemble d'aiguille comprenant au moins une aiguille à feutrer (209) agencée ou pouvant être agencée au niveau de l'extrémité distale de l'élément tubulaire allongé, dans lequel la au moins une aiguille à feutrer est mobile par rapport à l'élément tubulaire allongé,
et dans lequel la au moins une aiguille à feutrer est mobile avec un mouvement de va-et-vient, et
- un ensemble d'entraînement (204, 205, 210) agencé dans la lumière intérieure de l'élément tubulaire, dans lequel l'ensemble d'entraînement est raccordé, de manière opérationnelle, à l'ensemble d'aiguille pour déplacer la au moins une aiguille à feutrer avec le mouvement de va-et-vient, lorsque la au moins une aiguille à feutrer est positionnée au niveau de l'extrémité distale de sorte que le textile feutrable peut être fixé au niveau du site cible.

2. Dispositif de fixation selon la revendication 1, dans lequel l'ensemble d'entraînement est adapté pour entraîner le mouvement de va-et-vient avec une amplitude dans la plage de 2 à 25 mm, de préférence de 6 à 16 mm, de manière préférée entre toutes de 8 à 12 mm.

3. Dispositif de fixation selon l'une des revendications précédentes, dans lequel le dispositif de fixation comprend, de plus, le textile feutrable (108 ; 308 ; 408 ; 508) et dans lequel le textile feutrable (108 ; 308 ; 408 ; 508) est agencé ou peut être agencé de sorte qu'au moins une aiguille à feutrer (209) pénètre dans le textile feutrable avec le mouvement de va-et-vient.

4. Dispositif de fixation selon l'une des revendications précédentes, dans lequel la au moins une aiguille feutrable (209) pointe dans une direction distale de l'élément tubulaire allongé.

5. Dispositif de fixation selon l'une des revendications précédentes, dans lequel le mouvement de va-et-vient de la au moins une aiguille à feutrer est linéaire ou rotatif.

6. Dispositif de fixation selon l'une des revendications précédentes, dans lequel l'ensemble d'entraînement comprend un fil ou tube d'entraînement allongé (210) qui s'étend à partir d'un côté proximal de l'élément tubulaire allongé vers l'ensemble d'aiguille, et dans lequel le fil ou tube d'entraînement allongé est raccordé, de manière opérationnelle, à l'ensemble d'aiguille de sorte que le fil ou tube d'entraînement allongé provoque le mouvement de va-et-vient de la au moins une aiguille à feutrer.

7. Dispositif de fixation selon la revendication 6, dans lequel l'ensemble d'entraînement comprend un élément résilient (204) qui est agencé de sorte que l'élément résilient force la au moins une aiguille à feutrer à nouveau dans une position prédéterminée lorsque la au moins une aiguille à feutrer est actionnée par le fil ou tube d'entraînement allongé.

8. Dispositif de fixation selon les revendications 6 ou 7, dans lequel le fil ou tube d'entraînement allongé (210) est configuré pour transférer une force de traction et facultativement de compression le long de la direction allongée du fil ou tube d'entraînement allongé (210) à l'ensemble d'aiguille, de sorte que le fil d'entraînement (210) actionne le mouvement de va-et-vient de la au moins une aiguille à feutrer.

9. Dispositif de fixation selon l'une des revendications 6 à 8, dans lequel le fil ou tube d'entraînement allongé (210) comprend un revêtement à faible friction sur une surface externe, en particulier un revêtement en polytétrafluoroéthylène.

10. Dispositif de fixation selon l'une des revendications précédentes, dans lequel l'élément tubulaire allongé comprend au moins un élément de guidage (201) pour l'ensemble d'entraînement dans la lumière intérieure.

11. Dispositif de fixation selon l'une des revendications précédentes, comprenant le textile feutrable (308), dans lequel le textile feutrable a la forme d'une bande (508) ou d'une pièce (308).

12. Dispositif de fixation selon l'une des revendications précédentes, comprenant le textile, dans lequel le textile a une forme annulaire et/ou forme un anneau d'annuloplastie (805).

13. Dispositif de fixation selon l'une des revendications précédentes, dans lequel le dispositif de fixation comprend, de plus, un ensemble de support pour le textile feutrable, dans lequel l'ensemble de support comprend au moins un support (409 ; 510 ; 511) pour fixer le textile sur l'ensemble de support, dans lequel le textile est dans une position pliée.

14. Dispositif de fixation selon la revendication 13, comprenant une gaine externe tubulaire (402) avec une extrémité distale, dans lequel l'ensemble de support est mobile par rapport à la gaine externe et dans lequel, après avoir été expulsé de l'extrémité distale de la gaine externe, l'ensemble de support adopte une position expansée de sorte que le textile est déplié.

15. Ensemble comprenant un dispositif de fixation selon l'une des revendications précédentes, et un implant comprenant le textile feutrable.
